# DEMANDE DE BREVET EUROPEEN

(11) **EP 4 591 850 A1**
(43) Date de publication de la demande: **30.07.2025**
(21) Numéro de dépôt: 24020349.7
(22) Date de dépôt: 18.12.2024
(51) Int. Cl.: A61K 8/49, A61K 8/9789, A61Q 1/02, A61Q 1/04, A61Q 1/06, A61Q 1/08, A61Q 1/10, A61Q 3/02, A61K 8/35

(54) **MATIÈRE PREMIÈRE COLORANTE ISSUE DE PLANTES PARTICULIÈRES SELON UN PROCÉDÉ METTANT EN OEUVRE UNE EXTRACTION PARTICULIÈRE ET UN ENROBAGE PARTICULIER**

(30) Priorité: 02.01.2024 FR 2400018
(71) Demandeur: Le Rouge Français, 92140 Clamart (FR)
(72) Inventeur: Guillouzo, Laura, 13002 MARSEILLE (FR); Carpentier, Elodie, 92140 CLAMART (FR); Ghezaili, Salem, 92140 CLAMART (FR); Masmoudi, Yasmine, 13090 AIX EN PROVENCE (FR); Mouahid, Adil, 13127 VITROLLES (FR); Badens, Elisabeth, 13008 MARSEILLE (FR)

(57) **Abrégé**

Matière première colorante constituée d'un extrait colorant issu de la totalité ou d'une ou plusieurs parties d'une ou plusieurs plantes de la sous classe des Asteridées choisies parmi les Rubiacées et/ou les Bignoniacées à partir d'un procédé comprenant (a) une étape d'extraction par un fluide supercritique, puis (b) une étape d'enrobage par un ingrédient ou une composition à base d'acides gras libres. Composition comprenant cette matière colorante, utilisée en particulier dans le domaine cosmétique. Procédé de maquillage, notamment de la peau ou des lèvres ou des cils ou des ongles, mettant en œuvre une telle composition.

## Description

Dans de nombreux domaines pour apporter de la couleur à un substrat il est connu d'utiliser des matières premières colorantes qui sont soit des colorants solubles soit des pigments insolubles. Les espèces colorantes peuvent être d'origine naturelle ou synthétique. Beaucoup de colorants ou de pigments colorés d'origine synthétique posent des problèmes d'innocuité, notamment au niveau des risques de génotoxicité ou de potentiel sensibilisant. Par ailleurs ces colorants ou pigments synthétiques ne sont pas issus de sources renouvelables et leur empreinte carbone n'est pas favorable.

Dans les matières colorantes d'origine naturelle on utilise parfois des espèces d'origine minérale mais d'une part la palette des teintes obtensibles est limitée et d'autre part on peut se retrouver face à des problèmes de traces de métaux lourds.

Il est donc logique de se tourner vers les espèces colorantes issues du monde vivant et tout particulièrement pour des questions éthiques vers les espèces colorantes issues du monde végétal.

On se trouve alors confronté à différentes difficultés.

La première difficulté est relative aux concentrations relativement faibles en molécules colorantes actives présentes dans les végétaux. Il s'avère que souvent les extractions de ces molécules colorantes à partir des végétaux concernés sont réalisées avec de l'eau à pression atmosphérique éventuellement en chauffant en évitant les températures trop élevées pour éviter les risques de dégradation. Les rendements d'extraction sont alors assez faibles et les produits obtenus peu concentrés.

La seconde difficulté se situe au niveau des propriétés intrinsèques des espèces colorantes d'origine végétale. Beaucoup d'entre elles sont relativement fragiles notamment sur le plan de la stabilité face à l'action de composés chimiques ou à des variations de pH, ou sur le plan de la résistance à la lumière naturelle. Enfin certaines d'entre elles se dispersent difficilement dans les milieux de formulation.

Le troisième niveau se situe au niveau de propriétés connexes de l'extrait. En particulier très souvent l'extraction n'est pas univoque et on retrouve dans l'extrait final des composés gênants comme ceux conférant à l'extrait une mauvaise odeur.

Tout ce qui précède est vrai pour de nombreux domaines d'utilisation, par exemple dans le domaine alimentaire. C'est aussi particulièrement vrai pour le domaine de la cosmétique où existe un véritable besoin de modifier la couleur des différents substrats présents sur le corps humain.

Il a été proposé dans le document MY161184A de l'Université de Technologie Mara des nanocolorants avec des pigments obtenus à partir d'anthocyanes de fleurs et en-capsulés dans un second temps dans des microparticules de chitosane ou d'alginate. Outre le fait qu'ils s'agit de pigments végétaux particuliers et que l'extraction est réalisée par utilisation d'un solvant choisi parmi l'eau, les solvants organiques miscibles à l'eau ou leurs mélanges et ceci à pression atmosphérique, ce qui limite les rendements d'extraction, on notera que le chitosane est d'origine animale et que ses sources habituelles sont des crustacés ce qui peut induire des risques de sensibilisation chez l'être humain. Les alginates sont quant à eux issus des algues brunes. Ces alginates ont comme principaux défauts une sensibilité aux ions calcium (gélification hétérogène), des problèmes potentiels de coloration parasite et/ ou d'odeur résiduelle désagréable dans leur mise en œuvre.

Pour résoudre ces problèmes la demanderesse a proposé dans la demande FR3119989 (A1) des matières colorantes constituées d'un extrait colorant issu de la totalité ou d'une ou plusieurs parties d'une ou plusieurs plantes à partir d'un procédé comprenant les étapes suivantes:
- (a) une phase d'extraction comprenant au moins une extraction (aᵢ) avec action d'un ou plusieurs agents exogènes choisis parmi les solvants sous pression, les enzymes, les ondes radioélectriques de fréquences allant de 16 KHz à 300 GHz.
- (b) un enrobage de l'extrait issu de la phase d'extraction (a) avec un ou plusieurs agents issus d'une ou plusieurs plantes.

Dans les agents d'enrobage sont mentionnés parmi d'autres produits les corps gras sans plus de précisions. Les agents d'enrobage préférés sont choisis dans ce document parmi les polysaccharides ou les polypeptides dont les protéines et ce sont les seuls types d'agents illustrés dans les exemples.

Néanmoins les matières colorantes décrites dans ce document ne présentent pas des qualités optimales notamment pour la mise en œuvre dans la réalisation des compositions finies.

Dans la demande non publiée FR2300912, la demanderesse a proposé un procédé de préparation d'un extrait d'au moins une plante appartenant à la famille des Rubiacées comprenant les étapes suivantes:
(a) réduire en poudre la ou les plantes ou la ou les parties de plantes dont celle ou celles appartenant à la famille des Rubiacées pour obtenir après cette opération une poudre d'une granulométrie moyenne allant de 400 µm à 1500 µm.
(b) soumettre la poudre obtenue dans l'étape (a) à une extraction par le dioxyde de carbone supercritique à une pression supérieure ou égale à 30 MPa et à une température supérieure ou égale à 45°C.

Il est indiqué dans ce document que le procédé de préparation peut comprendre d'autres étapes telles qu'une étape d'enrobage ou d' encapsulation. Cet enrobage peut résulter d'un dépôt d'un ou plusieurs matériaux solides ou liquides sur l'extrait ou de l'inclusion de l'extrait dans une particule formée par un ou plusieurs matériaux. La nature chimique de l'agent d'enrobage n'est pas spécifiée.

Le document CN102558131 (A) de la société SUZHOU BAOZETANG PHARMA-CEUTICAL TECHNOLOGY CO LTD décrit un procédé d'extraction par fluide supercritique de garance, caractérisé en ce que: la poudre de garance est infiltrée avec une solution d'éthanol, mise dans une bouilloire d'extraction, le dioxyde de carbone supercritique étant utilisé comme agent d'extraction, et l'éthanol étant utilisé comme entraîneur pour extraire, suivi d'une décompression pour la séparation et la récupération, puis utilisation de la chromatographie sur colonne pour purifier et séparer l'extrait, dans lequel la pression d'extraction est de 25 à 45 MPa, la température est de 30 à 60 ° C et le temps d'extraction est de 1 à 5 heures, la pression pour l'analyse étant de 4 à 8 MPa; le remplissage de colonne chromatographique étant réalisé avec le gel de silice et l'éluant étant un solvant mixte éther-acétate d'éthyle de pétrole, d'éthanol ou de méthanol, la solution d'éthanol utilisée pour l'infiltration de la présente invention étant une solution d'éthanol avec un pourcentage volumique de 70 à 95 %, le rapport solide/ liquide étant de 1:3-8, et l'infiltration prenant de1 à 3 heures.

Aucun enrobage de l'extrait obtenu n'est mentionné.

Le document WO2015105407 (A1) est un document de la société SIRIM BERHAD relatif à un procédé de production d'une formulation nanolipidique utilisée pour le soin et/ou la réparation de la peau, caractérisé en ce que ledit procédé utilise une combinaison de lipide solide et d'un système de support lipidique liquide encapsulant un extrait par fluide supercritique de Curcuma Xanthorrhiza, dans lequel; lesdits lipides solides et liquides sont chauffés au-dessus de leur point de fusion et mélangés ensemble pour former une phase lipidique; ledit extrait fluide supercritique de Curcuma Xanthorrhiza est ensuite ajouté à ladite phase lipidique ; puis on ajoute une phase aqueuse chaude d'eau distillée et de tensioactif pour former un mélange dudit système de support lipidique; et on soumet ledit système de support lipidique à un processus d'homogénéisation à haute pression pour produire ladite formulation de nanolipides.

Dans ce document l'extrait de Curcuma Xanthorrhiza n'est pas spécifié comme un extrait colorant.

Par ailleurs les seuls lipides cités dans ce document sont la cire d'abeille, l'alcool cétylique plus l'huile d'abricot dans l'exemple 2.

Il existe donc toujours un besoin de mettre à disposition de nouvelles matières premières colorantes à partir d'un procédé qui permet de surmonter les inconvénients mentionnés précédemment.

A cet effet, la demanderesse préconise une matière première colorante constituée d'un extrait colorant issu de la totalité ou d'une ou plusieurs parties d'une ou plusieurs plantes de la sous classe des Asteridées choisies parmi les Rubiacées et/ou les Bignoniacées à partir d'un procédé comprenant (a) une étape d'extraction par un fluide supercritique, puis (b) une étape d'enrobage par un ingrédient ou une composition à base d'acides gras libres.

Outre son origine naturelle, la matière première colorante obtenue possède une efficacité maximalisée avec un maintien de cette efficacité dans le temps ou face à la lumière.De plus ces matières première présentent une bonne innocuité ainsi qu'une mise en œuvre plus aisée en formulation notamment en cosmétique.

Par extrait colorant de plante on entend au sens de la présente invention un extrait contenant un ou plusieurs colorants issus d'une ou plusieurs des plantes sélectionnées soit à partir d'une plante unique ou d'un mélange de plantes A.

La sous classe des Asteridées (ou Asteridae) est celle telle que définie par la classification de CONQUIST de 1981.

Les extraits colorants de plantes peuvent provenir de toutes les parties de la plante et en particulier de la plante entière ou des parties aériennes ou souterraines telles que des racines, des tiges ou des troncs et notamment des écorces, des feuilles, des fleurs, des graines et des fruits. Ils peuvent aussi être issus de sécrétions ou exsudats de plantes produits par les dites plantes, en particulier en réaction ou en prévention à une agression.

Les Rubiacées (Rubiaceae) sont une famille de plantes à fleurs. Elles appartiennent selon la classification de CONQUIST (1981) au règne Plantae, au sous règne Tracheobionta, à la division Magnoliophyta, à la classe Magnoliopsida, à la sous classe Asteridae, à l'ordre des Rubiales

Ce sont des arbres, des buissons, des lianes ou des plante herbacées largement répandus dans les régions froides, tempérées, subtropicales ou tropicales.

De préférence on utilisera dans l'invention des plantes de la famille des Rubiacées appartenant aux genres Galium, Asperula, Genipa, Gardenia ou Rubia. Encore plus préférentiellement on utilisera des plantes de la famille des Rubiacées appartenant au genre Rubia.

Encore plus préférentiellement les plantes de la famille des Rubiacées de l'invention sont choisies parmi les espèces suivantes: Rubia tinctorum, Rubia peregrina, Rubia cordifolia, Rubia fructicosa.

Les Bignoniacées (Bignoniaceae) sont aussi une famille de plantes à fleurs. Elles appartiennent selon la classification de CONQUIST (1981) au règne Plantae, au sous règne Tracheobionta, à la division Magnoliophyta, à la classe Magnoliopsida, à la sous classe Asteridae, à l'ordre des Scrophulariales. Ce sont des arbres, des arbustes, des lianes des régions tempérées à tropicales.

De préférence on utilisera dans l'invention des plantes de la famille des Bignoniacées appartenant aux genres Bignonia (ou Arrabidaea), Campsis, Catalpa, Radermachera. Encore plus préférentiellement on utilisera des plantes de la famille des Bignoniacées appartenant au genre Bignonia (ou Arrabidaea).

Le mélange de plantes A peut contenir outre des plantes appartenant aux familles des Rubiacées ou des Bignoniacées d'autres plantes additionnelles. Ces plantes additionnelles peuvent appartenir ou non au sous ordre des Astéridées.

Dans une variante préférée de l'invention le mélange de plantes A ne contient que des Rubiacées ou des Bignoniacées.Encore plus préférentiellement le mélange de plantes A ne contient que des Rubiacées.

De préférence, l'extrait préparé par le procédé de l'invention contient au moins un colorant de formule (I) 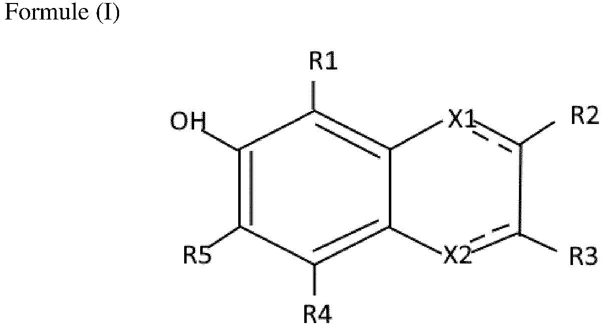 dans laquelle:
X1 désigne un atome d'oxygène chargé positivement ou un groupe carbonyle
X2 désigne un groupe carbonyle ou un groupe CH
R1 et R5 désignent indépendamment l'un de l'autre un atome d'hydrogène ou un groupe hydroxy
R4 désigne , un atome d'hydrogène ou un groupe hydroxy ou un groupe méthoxy
R3 désigne un atome d'hydrogène ou forme avec R2 et les deux atomes de carbone portant les radicaux R2 et R3 un cycle benzénique
R2 désigne un groupe 4' méthoxy phényle ou 4' hydroxy phényle ou 3',4' dihydroxy phényle ou forme avec R3 et les deux atomes de carbone portant les radicaux R2 et R3 un cycle benzénique
Plus préférentiellement le colorant de formule ci dessus est choisi parmi l'alizarine, la purpurine, la carajurine, la carajurone et la 3'hydroxy carajurone.
Encore plus préférentiellement le colorant de formule ci dessus est choisi parmi l'alizarine et la purpurine.

L'étape (a) du procédé de l'invention est une extraction par un fluide supercritique. Par fluide supercritique on entend un composé maintenu au delà de son point critique, c'est à dire chauffé au delà de sa température critique lorsqu'il est comprimé au dessus de sa pression critique. Le domaine d'existence d'un fluide supercritique est défini par le diagramme de phases pression-température du composé correspondant, le point critique étant le point où s'arrête la courbe d'équilibre liquide-gaz. Les fluides supercritiques ont un comportement pseudo-liquide.

Par pseudo-liquide on entend l'état d'une substance présentant une densité intermédiaire entre celle de la phase liquide et celle de la phase gazeuse. Ces fluides supercritiques allient donc densité proche d'un liquide et diffusivité d'un gaz.

De préférence le fluide supercritique utilisé est le dioxyde de carbone supercritique pour lequel le point critique est défini par la température critique de 31°C et la pression critique de 73,8 bars (soit 7,38 MPa , 1 MPa étant équivalent à 10⁶ Pa et 1 bar à 10⁵ Pa).

L'extraction de l'étape (a) se fait à une pression allant de préférence de 10 MPa à 250 MPa, mieux de 15 MPa à 200 MPa.

L'extraction de l'étape (a) peut s'effectuer à une température allant de préférence de 31°C à 200 °C, plus préférentiellement de 41°C à 160°C, mieux de 45°C à 100°C, encore mieux de 50° C à 80 °C.

De préférence le durée de l'étape (a) est inférieure ou égale à 5 heures. Mieux elle varie de 30 minutes à 5 heures, encore mieux de 1 heure à 4 heures.

Le procédé de l'invention comprend une étape (b) d'enrobage de l'extrait issu de l'étape (a) par un ingrédient ou une composition à base d'acides gras libres.

Par agent d'enrobage ou agent d'encapsulation au sens de la présente invention on entend un composé permettant d'isoler au moins partiellement l'extrait du matériau issu du procédé de l'invention du milieu environnant, dans les conditions de stockage à température ambiante et à pression atmosphérique.

Par ingrédient ou composition à bases d'acides gras libres on entend un ingrédient ou une composition comprenant au moins 50% en poids d'acides gras libres.

De préférence l'agent d'enrobage comprend au moins 75% en poids et plus préférentiellement au moins 90% en poids d'acides gras libres.

Par acide gras libre on entend un acide carboxylique possédant une chaîne grasse hydrocarbonée comportant de de 7 à 40 atomes de carbone, non salifié et non estérifié.

De préférence les acides gras utilisables dans l'étape (b) sont des acides gras linéaires ou ramifiés saturés possédant une température de fusion à pression atmosphérique allant de 75°C à 85°C. Encore plus préférentiellement les acides gras utilisables dans l'étape (b) sont des acides gras linéaires possédant une température de fusion à pression atmosphérique allant de 75°C à 85°C.

Préférentiellement les acides gras sont choisis parmi l'acide béhénique, l'acide arachidique ou leurs mélanges.

L'enrobage peut résulter d'un dépôt d'un ou plusieurs ingrédients à base d'acides gras sur l'extrait ou de l'inclusion de l'extrait dans une particule formée par un ou plusieurs ingrédients à base d'acides gras.Les particules évoquées sont de préférences des microparticules. Si elles sont creuses on parlera de microencapsulation. Si elles sont pleines avec des espaces d'inclusion on parlera de microsphères.

Le dépôt simple peut se faire par des techniques de pulvérisation, d'attraction, de friction ou de cristallisation.

Les principales technique d'encapsulation utilisables sont l'atomisation (spray drying), l'extrusion, la coacervation, la formation de liposomes, la gélification ionique, la gélification thermique, la lyophilisation, le lit-fluidisé, l'émulsification via des émulsions multiples.

De préférence on utilisera un enrobage mettant en œuvre un fluide supercritique, de préférence le même que celui utilisé dans l'étape (a), encore plus préférentiellement le dioxyde de carbone supercritique. Les conditions de pression et de température par cet enrobage en présence d'un fluide supercritique peuvent être identiques ou différentes de celles de l'étape (a). Préférentiellement on opérera à une pression inférieure à celle de l'étape (a).

Encore plus préférentiellement on opérera avec une combinaison de pression et de température pour laquelle le ou les acides gras libres sont à l'état liquide.

Dans la pratique l'étape d'extraction (a) peut se faire dans un autoclave (1) avec entraînement de l'extrait par le fluide supercritique vers un autoclave (2) et dans lequel l'enrobage est réalisé toujours en présence du fluide supercritique. L'ingrédient à base d'acides gras est de préférence introduit dans l'autoclave (2) avant l'extraction. On peut aussi envisager de l'introduire en cours ou après l'extraction. L'étape d'enrobage en elle même est de préférence réalisée après la fin de l'extraction. Le contenu de l'autoclave (2) est soumis lors de l'enrobage à des conditions de pression et de température identiques ou différentes de celles de l'étape (a). L'étape d'enrobage (b) se termine alors après un certain temps de contact (une heure par exemple) par un retour du milieu à température ambiante et à pression atmosphérique.

Le procédé d'obtention des matières colorantes de l'invention peut comprendre en plus d'autres étapes que les étapes d'extraction et d'enrobage précédemment décrites.

Parmi ces étapes complémentaires on peut citer celles mettant en œuvre une ou plusieurs des opérations suivantes : broyage, filtration, évaporation ou concentration, tamisage, séchage, lyophilisation.

Certaines de ces autres étapes peuvent prendre place avant ou après les phases d'extraction et d'enrobage.

Les matières premières colorantes de l'invention sont mises en œuvre dans des compositions.

Ces compositions contiennent une ou plusieurs matières premières colorantes selon l'invention.

La concentration en matières premières colorantes de l'invention dans ces compositions peut varier de 0,001% à 60% en poids, plus préférentiellement de 0,1% à 50% en poids, mieux de 0,5% à 40% en poids, encore mieux de 1% à 35% en poids par rapport au poids total de la composition.

Les matières colorants de l'invention peuvent être complètement solubilisées ou de préférence dispersées dans la composition les contenant. Par dispersée on entend que la matière colorante est insolubilisée ou pas complètement solubilisée dans la composition à température ambiante et à pression atmosphérique.

De par son enrobage particulier la mise en œuvre des matières colorantes de l'invention dans les compositions finales est facilitée.

Il est possible d'ajouter aux matières colorantes de l'invention dans les compositions selon l'invention d'autres matières colorantes soit solubles dans l'eau, soit insolubles dans l'eau à température ambiante et à pression atmosphérique. Ces matières colorantes additionnelles peuvent être des colorants naturels dont les colorants issus de plantes mais obtenus par un procédé différent de celui de l'invention. Ces matières colorantes additionnelles peuvent être aussi des pigments ou des colorants solubles organiques de synthèse ou des pigments minéraux.

De préférence, les matières colorantes des compositions selon l'invention sont toutes d'origine végétale.

Les compositions de l'invention peuvent être anhydres ou aqueuses.

Par anhydre on entend au sens de la présente invention une composition contenant à température ambiante et à pression atmosphérique moins de 5% d'eau en poids, encore mieux moins de 1% d'eau en poids par rapport au poids total de la composition et préférentiellement ne contenant pas d'eau, en particulier d'eau ajoutée.

Par aqueuse on entend au sens de la présente invention une composition contenant à température ambiante et à pression atmosphérique de 5% à 99,01% d'eau en poids par rapport au poids total de la composition.

De préférence si elle est aqueuse, la composition comprend à température ambiante et à pression atmosphérique de 25% à 98% d'eau en poids, mieux de 40% à 95% d'eau en poids par rapport au poids total de la composition.

Dans une variante préférée de l'invention les compositions cosmétiques sont anhydres.

Les compositions selon l'invention peuvent se présenter sous forme de poudres, de solides compacts tels que des sticks, de liquides, de gels, d'émulsions simples ou multiples, de mousses aérosols.

Les compositions selon l'invention contiennent de préférence un ou plusieurs corps gras additionnels.

Le ou les corps gras sont choisis parmi les huiles ou les cires.

Les compositions de l'invention peuvent contenir une ou plusieurs huiles.

Par huile on entend au sens de la présente invention un composé non miscible à l'eau liquide à température ambiante et à pression atmosphérique.

Les huiles peuvent être polaires ou apolaires, volatiles ou non volatiles (une huile est dite non volatile quand sa pression de vapeur est inférieure à 0,02 mm de mercure à pression atmosphérique et à température ambiante).

Les huiles peuvent être hydrocarbonées, d'origine végétale, minérale ou synthétique. Elles peuvent être aussi siliconées ou fluorées.

Les huiles siliconées peuvent être des polydiméthylsiloxanes linéaires ou ramifiés ou cycliques porteurs éventuellement de groupes aryle et en particulier phényle.

Les huiles hydrocarbonées apolaires sont choisies de préférence parmi l'huile de paraffine, les isoparaffines (isododécane, isodécane), les alcanes linéaires (n-dodécane), le squalane, l'eicosane, les polybutylènes ou les polyisobutylènes hydrogénés ou non, les polydécènes hydrogénés ou non.

Les huiles polaires sont de préférence hydrocarbonées. Elles peuvent être choisies parmi les alcools gras ramifiés ou insaturés comme l'alcool oléique et l'octyldodécanol. Elles sont de préférence choisies parmi les triglycérides d'origine végétale ou synthétique et les esters d'acides gras autres que les triglycérides.

Parmi les huiles d'origine végétale on peut notamment citer l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyaux d'abricot, l'huile d'olive, l'huile d'onagre, l'huile de palme, l'huile de passiflore, l'huile de pépins de raisin, l'huile de ricin, l'huile de rosier, l'huile de seigle, l'huile de sésame, l'huile de son de riz, l'huile de soja et l'huile de tournesol.

De préférence l'huile ou les huiles est ou sont choisies parmi les huiles végétales.

Les compositions selon l'invention peuvent contenir une ou plusieurs cires.

Par cire on entend au sens de la présente invention un composé lipophile solide à température ambiante et à pression atmosphérique, à changement d'état solide/liquide réversible, ayant un point de fusion supérieur à 30°C et de préférence inférieur à 120°C. Le point de fusion de la cire peut être mesuré à l'aide d'un calorimètre à balayage différentiel (DSC), par exemple celui proposé par la société METTLER sous la référence DSC 30.

Les cires peuvent être hydrocarbonées, siliconées ou fluorées et être d'origine naturelle minérale, animale, végétale ou d'origine synthétique. Elles peuvent être polaires ou apolaires.

Comme cires apolaires on peut citer les cires microcristallines, les cires de paraffines, l'ozokérite, les cires de polyéthylènes.

Les cires polaires sont de préférence hydrocarbonées et encore plus préférentiellement choisies parmi les cires esters comprenant au moins une fonction ester et les cires alcools comprenant au moins une fonction alcool.

Les cires esters sont de préférence choisies parmi les esters d'acide(s) gras et/ou d'alcools gras sous forme solide à température ambiante et à pression atmosphérique. Ces cires esters peuvent être issues d'huiles animales ou végétales hydrogénées.

Les cires alcools ont de préférence choisies parmi les alcools gras sous forme solide à température ambiante et à pression atmosphérique tels que l'alcool cétylique ou l'alcool cétylstéarylique.

Les cires d'origine naturelle sont de préférence choisies parmi la cire de carnauba, la cire de candelilla, la cire de riz, la cire d'ouricury, la cire d'alfa, la cire de liège, la cire de canne à sucre, la cire de mimosa, la cire d'orange, la cire de laurier, la cire de pomme, la cire de jojoba, la cire de rose, la cire de tournesol, la cire d'olive, la cire de soja, la cire d'acacia, la cire de citron, la cire de macadamia, la cire d'abricot, la cire d'olive, la cire d'avocat, la cire de pêche, la cire de colza, la cire de coco, la cire de palme.

De préférence la ou les cires est ou sont choisies parmi les cires végétales.

Mieux les compositions de l'invention ne contiennent à titre de cires que des cires végétales.

Plus préférentiellement la ou les cires est ou sont choisies parmi la cire de carnauba, la cire de candelilla et la cire de riz.

De préférence les compositions selon l'invention contiennent au moins une huile végétale et/ou au moins une cire végétale.

Dans les compositions selon l'invention la teneur en corps gras peut varier de 1% à 98% en poids, mieux de 10% à 95% en poids, encore mieux de 20% à 95% en poids par rapport au poids total de la composition.

Les compositions selon l'invention peuvent comprendre un ou plusieurs agents tensioactifs choisis parmi les agents tensioactifs anioniques, non-ioniques, cationiques ou amphotères.

Les compositions selon l'invention peuvent en outre comprendre un ou plusieurs additifs classiques bien connus dans la technique.

A titre d'exemples d'additifs utilisables selon l'invention, on peut citer les polymères associatifs ou non associatifs, ioniques ou non-ioniques et en particulier les polymères épaississants, les épaississants minéraux, les filtres UVA et/ou UVB, les agents émollients, les solvants, les vitamines, les pro-vitamines, les acides aminés, les parfums ou arômes, les agents peptisants, les agents conservateurs, les agents antioxydants, les agents alcalinisants ou acidifiants (notamment pour réguler le pH des compositions aqueuses ou pour salifier les ingrédients), les agents fixateurs de couleur, les agents matifiants comme le talc, le kaolin, la silice ou le dioxyde de titane.

Les compositions cosmétiques peuvent en plus des ingrédients ci dessus contenir des agents spécifiques à ce domaine comme des agents antirides, des agents hydratants des matières kératiniques, des agents adoucissants pour la peau, des agents kératolytiques, des agents anti-sébum.

Les domaines d'utilisation des matières premières colorantes selon l'invention peuvent être très variés. Il peut s'agir par exemple d'une mise en œuvre dans des produits alimentaires ( boissons, produits laitiers, glaces, desserts divers..), dans des produits ménagers ( lessives solides ou liquides, nettoyants ménagers, désodorisants, produits anti-insectes...), dans des matériaux industriels ( plastiques, peintures, vernis, lasures...), dans des produits pour impression (encres), dans l'industrie textile notamment pour les opérations de teinture, dans la parapharmacie et dans les produits cosmétiques.

Encore plus préférentiellement, les matières premières colorantes selon l'invention sont utilisées pour modifier la couleur des substrats sur lesquels on les dépose.

Préférentiellement les matières colorantes selon l'invention sont utilisées dans le domaine cosmétique, encore mieux dans des compositions de maquillage.

Par composition pour le maquillage, on entend au sens de la présente invention une composition apportant de la couleur au substrat anatomique sur lequel elle est appliquée.

Ces substrats anatomiques peuvent être les cheveux, la cavité buccodentaire, la peau, les lèvres, les ongles, les cils, les sourcils.

De préférence les substrats cosmétiques sur lesquels sont appliquées les matières premières colorantes de l'invention sont la peau, les lèvres, les ongles et les cils. Les matières premières colorantes de l'invention sont alors préférentiellement utilisées dans des compositions pour le maquillage de la peau, ou des lèvres, ou des ongles, ou des cils.
et se présentant de préférence sous la forme d'un rouge à lèvres, d'un crayon pour les lèvres, d'un fluide à lèvres, d'un mascara, d'un fond de teint, d'un fard à joues, d'une ombre à paupières, d'un eye liner.

Pour optimiser la couvrance des substrats par les matières premières colorantes de l'invention on peut incorporer dans les compositions de l'invention un ou plusieurs composés solides lamellaires insolubles dans l'eau et non siliciés qui peuvent être choisis parmi la guanine, l'oxyde de zinc et les stéarates de métaux divalents ( notamment stéarate de zinc ou de magnésium) ou leurs mélanges.

Par composé insoluble dans l'eau, on entend au sens de la présente invention un composé dont la solubilité dans l'eau est inférieure à 0,25% (0,25g pour 100m1), de préférence inférieure à 0,05%, mieux inférieure à 0,005% à température ambiante (de préférence 25°C) et à pression atmosphérique (1,013x10⁵ Pa).

Par composé lamellaire on entend au sens de la présente invention que le composé à température ambiante et à pression atmosphérique présente un arrangement des cristaux en feuillets ou plaquettes.

Par composé non silicié on entend au sens de la présente invention un composé ne comprenant pas d'atome de silicium dans sa formule chimique.

Par composé solide on entend qu'à température ambiante et à pression atmosphérique le composé lamellaire insoluble dans l'eau se présente se présente sous forme d'un solide, cristallisé ou amorphe.

Les compositions de maquillage citées peuvent notamment, de manière non limitative, se présenter sous forme de rouges à lèvres, de crayons pour les lèvres, de fluides à lèvres, de mascaras, de fonds de teint, de fards à joues, d'ombres à paupières, d'eye liners.

L'invention couvre également un procédé de maquillage de la peau ou des lèvres ou des cils ou des ongles, de préférence de la peau ou des lèvres , et encore plus préférentiellement des lèvres consistant à appliquer sur le substrat anatomique (peau ou lèvres ou cils ou ongles) une composition cosmétique comprenant une ou plusieurs matières colorantes selon l'invention telles que décrites ci dessus, sans rinçage subséquent.

### [Exemples]

Les exemples ci-dessous illustrent l'invention sans toutefois en limiter la portée. Exemple 1

### Exemple 1

30g de racines de garance de l'espèce Rubia tinctorum sont séchées, hachées et broyées finement pour obtenir une poudre d'une granulométrie entre 500 µm et 800 µm introduite dans un autoclave (I). La poudre obtenue est soumise à une extraction par le dioxyde de carbone supercritique à une pression de 20 MPa et à une température de 60°C pendant 1,5 heures avec un flux de dioxyde de carbone de 22,9 g de dioxyde de carbone par minute ce qui correspond à un rapport CO2/biomasse de 69.

L'extrait A est recueilli dans un autoclave (1) soumis pendant l'extraction à une pression de 5 MPa et à une température de 25°C et dans lequel on a introduit avant l'extraction 2,7 g d'acide béhénique. On soumet alors après l'étape d'extraction le contenu de l'autoclave (2) à un flux de dioxyde de carbone à 15 MPa et à une température de 65°C pendant 1 heure. On récupère in fine après retour à pression atmosphérique et à température ambiante un extrait enrobé A1 stable et très peu sensible à l'environnement extérieur.

Cet extrait A1 est aisément introduit grâce à son enrobage particulier dans la composition suivante selon le tableau 1. Cette composition se présente sous la forme d'un bâton de rouge à lèvres.

**[Tableaux1]**

| Ingrédient | Quantité en g pour 100g de composition |
|---|---|
| Extrait colorant enrobé A1 | 6 |
| Oxyde de zinc | 14,4 |
| Stéarate de zinc | 1,6 |
| Huile de ricin | 19,25 |
| Huile de tournesol | 32,27 |
| Cire de Carnauba | 18 |
| Beurre de karité | 5,6 |
| Alun de potassium | 1,83 |
| Carbonate de sodium | 0,87 |
| Tocopherol | 0,18 |

### Exemple 2

Cet exemple est réalisé dans les mêmes conditions que celles de l'exemple 1 en substituant poids pour poids les 30g de racines de garance de l'espèce Rubia tinctorum par 30g de feuilles et tiges d' Arrabidaea chica (ou Bignonia chica). L'extraction conduit à un extrait B qui après enrobage fournit un extrait enrobé B1 lui aussi stable, très peu sensible à l'environnement extérieur et aisément formulable notamment dans les compositions de bâton de rouge à lèvres comme celle de l'exemple 1.

## Revendications

1. Matière première colorante constituée d'un extrait colorant issu de la totalité ou d'une ou plusieurs parties d'une ou plusieurs plantes de la sous classe des Asteridées choisies parmi les Rubiacées et/ou les Bignoniacées à partir d'un procédé comprenant (a) une étape d'extraction par un fluide supercritique, puis (b) une étape d'enrobage par un ingrédient ou une composition à base d'acides gras libres.

2. Matière première colorante selon la revendication 1 pour laquelle l'extraction (aᵢ) est réalisée avec le dioxyde de carbone supercritique.

3. Matière première colorante selon les revendications 1 et 2 pour laquelle le fluide supercritique lors de l'extraction (aᵢ) est soumis à une pression allant de 10 MPa à 250 MPa, mieux de 15 MPa à 200 MPa.

4. Matière première colorante selon l'une quelconque des revendications précédentes pour laquelle l'extraction (aᵢ) est réalisée à une température allant de 31°C à 200 °C, plus préférentiellement de 41°C à 160°C, mieux de 45°C à 100°C, encore mieux de 50° C à 80 °C.

5. Matière première colorante selon l'une quelconque des revendications précédentes pour laquelle les acides gras utilisables dans l'étape (b) sont des acides gras linéaires ou ramifiés saturés possédant une température de fusion à pression atmosphérique allant de 75°C à 85°C et plus préférentiellement des acides gras linéaires possédant une température de fusion à pression atmosphérique allant de 75°C à 85°C.

6. Matière première colorante selon l'une quelconque des revendications précédentes pour laquelle les acides gras utilisables dans l'étape (b) sont choisis parmi l'acide béhénique ou l'acide arachidonique ou leurs mélanges.

7. Matière première colorante selon l'une quelconque des revendications précédentes pour laquelle l'étape d'enrobage (b) se fait en présence d'un fluide supercritique, de préférence en présence de dioxyde de carbone supercritique.

8. Matière première colorante selon l'une quelconque des revendications précédentes pour laquelle l'étape d'enrobage (b) se fait à une combinaison de pression et de température pour laquelle le ou les acides gras libres sont à l'état liquide.

9. Matière première colorante selon l'une quelconque des revendications précédentes pour laquelle les plantes de la famille des Rubiacées appartiennent aux genres Galium, Asperula, Genipa, Gardenia ou Rubia et plus préférentiellement au genre Rubia.

10. Matière première colorante selon l'une quelconque des revendications précédentes pour laquelle les plantes de la famille des des Bignoniacées appartiennent aux genres Bignonia (ou Arrabidaea), Campsis, Catalpa, Radermachera et plus préférentiellement au genre Bignonia (ou Arrabidaea).

11. Matière première colorante selon l'une quelconque des revendications précédentes contenant au moins un colorant de formule (I). 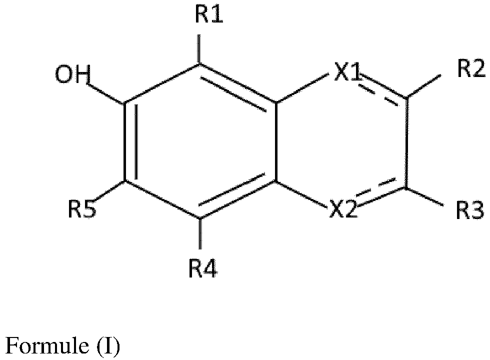 dans laquelle:
X1 désigne un atome d'oxygène chargé positivement ou un groupe carbonyle
X2 désigne un groupe carbonyle ou un groupe CH R1 et R5 désignent indépendamment l'un de l'autre un atome d'hydrogène ou un groupe hydroxy
R4 désigne , un atome d'hydrogène ou un groupe hydroxy ou un groupe méthoxy
R3 désigne un atome d'hydrogène ou forme avec R2 et les deux atomes de carbone portant les radicaux R2 et R3 un cycle benzénique
R2 désigne un groupe 4' méthoxy phényle ou 4' hydroxy phényle ou 3',4' dihydroxy phényle ou forme avec R3 et les deux atomes de carbone portant les radicaux R2 et R3 un cycle benzénique

12. Matière première colorante selon l'une quelconque des revendications précédentes contenant au moins un colorant choisi parmi l'alizarine, la purpurine, la carajurine, la carajurone et la 3'hydroxy carajurone et plus préférentiellement parmi l'alizarine et la purpurine.

13. Composition comprenant au moins une matière première colorante selon l'une quelconque des revendications 1 à 12 , de préférence dans une concentration allant de 0,001% à 60% en poids, plus préférentiellement de 0,1% à 50% en poids, mieux de 0,5% à 40% en poids, encore mieux de 1% à 35% en poids par rapport au poids total de la composition.

14. Composition selon la revendications 13 destinée au maquillage de la peau, ou des lèvres, ou des ongles, ou des cils et se présentant de préférence sous la forme d'un rouge à lèvres, d'un crayon pour les lèvres, d'un fluide à lèvres, d'un mascara, d'un fond de teint, d'un fard à joues, d'une ombre à paupières, d'un eye liner.

15. Procédé de maquillage de la peau ou des lèvres ou des cils ou des ongles, de préférence de la peau ou des lèvres, et encore plus préférentiellement des lèvres consistant à appliquer sur le substrat anatomique une composition selon l'une quelconque des revendications 13 et 14 sans rinçage subséquent.
